# DEMANDE DE BREVET EUROPEEN

(11) **EP 4 176 867 A1**
(43) Date de publication de la demande: **10.05.2023**
(21) Numéro de dépôt: 21206259.0
(22) Date de dépôt: 03.11.2021
(51) Int. Cl.: A61K 8/9789, A61K 36/68, A61P 17/00, A61Q 19/08

(54) **EXTRAIT DE PLANTE ET SON UTILISATION DANS DES COMPOSITIONS À USAGE TOPIQUE**

(71) Demandeur: Term Helvet SA, 6302 Zug (CH)
(72) Inventeur: SAURAT, Jean-Hilaire, 1206 GENÈVE (CH)
(74) Mandataire: Moinas & Savoye SARL

(57) **Abrégé**

Un extrait de plantes du genre *Picrorhiza,* choisies parmi *Picrorhiza kurroa, Picrorhiza scrophulariiflora* et *Neopicrorhiza scrophulariiflora* et leurs mélanges, obtenu par extraction par un solvant organique d'un hydrolysat aqueux acide d'éléments de la partie souterraine desdites plantes, lesdits éléments étant choisis parmi les rhizomes et les racines. Utilisation cosmétique ou dermatologique d'un tel extrait, dans une formulation topique, pour traiter un vieillissement normal ou pathologique de la peau.

## Description

### Domaine technique de l'invention

L'invention concerne un nouvel extrait de plante et son utilisation dans des compositions à usage topique.

L'invention trouve des applications dans le domaine cosmétique sous forme de compositions à usage topique, destinées à s'opposer aux signes visibles du vieillissement de la peau. L'invention trouve des applications dans le domaine dermatologique sous forme de compositions à usage topique, destinées à traiter des manifestations des premiers stades de la dermatoporose.

### Etat de la technique antérieure

Le vieillissement de la peau est l'un des signes les plus visibles du processus normal d'involution humaine. Les compositions cosmétiques à usage topique destinées à prévenir un vieillissement prématuré de la peau, photoinduit ou non, ont pour objectifs de s'opposer à l'amincissement des tissus cutanés, de diminuer les rides, de favoriser l'activité cellulaire épidermique et dermique, de raffermir la peau en améliorant son élasticité et/ou son état d'hydratation.

A titre d'exemple, on peut citer des compositions incorporant des rétinoïdes tels que le rétinal, les vitamines C ou E, ou encore l'extrait de *Centella asiatica* contenant des saponines triterpéniques, l'asiaticoside et le madécassoside.

Malgré l'existence de nombreuses préparations cosmétiques sur le marché, il demeure un besoin de développer d'autres compositions à usage topique destinées à s'opposer aux manifestations du vieillissement de la peau, soit en agissant sur un facteur spécifique de vieillissement, soit sur un syndrome plus global.

Cependant, associé à un âge avancé, ainsi qu'à d'autres facteurs tels que l'usage de traitements à bases de corticostéroïdes, un syndrome d'insuffisance cutanée fonctionnelle et chronique peut apparaître, qui dépasse largement les problèmes d'apparence mentionnés ci-dessus. J-H. Saurat a proposé le terme holistique « Dermatoporose », dans les documents [1] et [2], pour couvrir l'ensemble des manifestations de ce syndrome.

En résumant l'évolution de cette affection, et selon les publications traitant de cette affection, on distingue usuellement quatre stades :
Stade 1 : fort amincissement de la peau avec apparition de purpura sénile, suivi de pseudo-cicatrices stellaires.
Stade 2 : petites lacérations cutanées.
Stade 3 : augmentation des lacérations cutanées.
Stade 4 : apparition d'hématomes disséquants.

Les documents [1] à [8] cités dans la bibliographie ci-annexée traitent de ce sujet en détails.

Pour contrecarrer les premières manifestations du stade 1, on a proposé l'utilisation de hyaluronate. Le hyaluronate est synthétisé dans la peau normale par les fibroblastes dermiques et les kératinocytes épidermiques. Il favorise la retenue d'eau dans le tissu conjonctif de la peau et le maintien de l'élasticité de celle-ci. Sa synthèse diminue avec l'âge. L'administration topique de formulations à base de fragments de hyaluronate traversant la barrière cutanée permet d'augmenter la quantité d'hyaluronate présente *in situ,* contribuant au maintien de l'état d'hydratation de la peau et donc de raffermir celle-ci.

Le document [9] propose de traiter l'atrophie du tissu cutané par des préparations topiques associant des fragments spécifiques de hyaluronate et un rétinoïde. Toutefois, les premiers peuvent être difficiles à préparer dans leur poids moléculaire idéal et les seconds sont irritants.

Le document [10] enseigne l'utilisation de ligands agonistes des récepteurs AhR des tissus cutanés en vue de soigner diverses affections de ceux-ci. Ce document propose en particulier l'utilisation du L-tryptophane ou de certains de ses dérivés à durée de vie courte, c'est-à-dire métabolisables par des enzymes de phase I, pour traiter des manifestations de la dermatoporose. Le contenu de ce document est incorporé ici par cette référence [10].

Malgré l'existence de nombreuses préparations sur le marché, il demeure un besoin de développer d'autres compositions à usage topique destinées à prévenir ou traiter ce vieillissement pathologique de la peau, qu'il soit intrinsèque ou induit par les corticostéroïdes, en agissant sur un facteur spécifique de vieillissement, ou sur un syndrome plus global.

Le nom scientifique *Picrorhiza kurroa* Royle désigne une plante du genre taxonomique *Picrorhiza,* lequel fait partie de la famille des scrofulariacées. Cette plante pousse dans l'Himalaya du Kashmir au Sikkim entre 2700 et 4500 m d'altitude. Elle est l'une des plus anciennes plantes médicinales de la médecine ayurvédique, connue sous les noms vernaculaires locaux kutki ou katuki. La partie souterraine de la plante, à savoir son rhizome et ses racines, est utilisée traditionnellement dans la médecine ayurvédique en administration *per os* pour traiter divers problèmes digestifs (référence [11]).

Les composants essentiels du rhizome et des racines de cette plante étant dans l'ensemble faiblement solubles dans l'eau et certains d'entre eux extrêmement amers, la partie souterraine de la plante est usuellement utilisée sous forme séchée, broyée en poudre et encapsulée pour une administration *per os* en gélules, plutôt qu'en infusion. On a également proposé de l'administrer sous forme de poudre mélangée à du miel.

Des usages sont également cités pour traiter les piqûres de scorpion, l'eczéma et le vitiligo et ce également en administration *per os.*

### Objectifs de l'invention

L'invention a pour but de proposer des compositions à usage topique, destinées à traiter, chez l'Homme, des manifestations du vieillissement cutané tels que décrites ci-dessus.

### Solution selon l'invention.

L'invention a pour objet un nouvel extrait d'une plante du genre *Picrorhiza,* à savoir un extrait obtenu par extraction par un solvant organique à partir d'un hydrolysat aqueux acide d'éléments de la partie souterraine de ladite plante, choisis parmi son rhizome et ses racines. Les plantes du genre *Picrorhiza,* envisagées dans le cadre de l'invention, sont en particulier choisies parmi *Picrorhiza kurroa, Picrorhiza scrophulariiflora* et *Neopicrorhiza scrophulariiflora* et leurs mélanges.

L'invention a en particulier pour objet un nouvel «extrait d'hydrolysat de *P. kurroa*»*,* cette expression correspondant dans la suite de ce texte à un extrait, au moyen d'un solvant organique, d'un hydrolysat de racines et/ou de rhizome de la plante *Picrorhiza kurroa,* enrichi en composés monophénoliques provenant de l'hydrolyse acide de glycosides présents dans la plante. Ces composés monophénoliques sont essentiellement des aglycones des glycosides des racines et du rhizome de *Picrorhiza kurroa.*

L'invention a également pour objet l'utilisation d'un tel extrait en cosmétique, dans une formulation topique, pour obvier aux signes visibles de vieillissement de la peau. Cette utilisation de *Picrorhiza kurroa,* et en particulier de l'extrait résultant du procédé d'extraction de cette plante selon l'invention pour améliorer l'état de la peau lors des premiers signes de vieillissement cutané, constitue une innovation par rapport aux utilisations connues dans l'art antérieur.

L'invention a également pour objet l'utilisation d'un tel extrait dans une formulation topique pour contrecarrer les manifestations de vieillissement pathologiques de la peau décrites ci-dessus. Cette utilisation de *Picrorhiza kurroa,* et en particulier de l'extrait résultant du procédé d'extraction de cette plante selon l'invention, pour améliorer l'état de la peau lors des premiers stades de dermatoporose, constitue une innovation par rapport aux utilisations connues dans l'art antérieur.

Selon l'invention, un extrait est défini par les revendications 1 et 12.

Différents modes de réalisation d'extrait sont définis par les revendications 2 et 4.

Selon l'invention, un mélange est défini par la revendication 5.

Selon l'invention, une composition est définie par les revendications 6 et 12.

Différents modes de réalisation de composition sont définis par les revendications 7 à 10.

Selon l'invention, une utilisation est définie par la revendication 11.

Selon l'invention, un procédé d'obtention d'un extrait est défini par la revendication 13.

D'autres caractéristiques de l'invention apparaîtront à l'homme du métier dans la description ci-dessous d'un mode d'exécution et des figures accompagnantes.

### Brève description des figures.

Un mode d'exécution de l'invention est décrit ci-dessous en se référant aux figures :
- La figure 1A est un chromatogramme d'un extrait de *Picrorhiza kurroa* obtenu sans hydrolyse acide,
- La figure 1B est un chromatogramme d'un extrait de *Picrorhiza kurroa* obtenu suite à une hydrolyse acide, selon l'invention.
- La figure 2 montre la formation de filopodes dans quatre cultures *in vitro* ayant subi des traitement différents.

### Description détaillée de l'invention.

Les espèces chimiques recherchées dans le cadre de l'invention sont notamment les aglycones de glycosides produits par la plante. Leur utilisation dans une formulation topique implique de les inclure sous forme d'aglycones libres car la peau n'hydrolyse pas les glycosides, et de plus, ceux-ci étant polaires, ne pénètrent pas dans la peau.

La matière première utilisée est une poudre obtenue à partir du broyage des racines et du rhizome de la plante *Picrorhiza kurroa,* ou de l'une des plantes *Picrorhiza scrophulariiflora,* ou *Neopicrorhiza scrophulariiflora,* ou encore d'un mélange de celles-ci.

Cette poudre est mise en suspension dans une solution aqueuse d'un acide pour réaliser l'hydrolyse des glycosides présents dans la poudre. L'acide chlorhydrique dilué, à une concentration de l'ordre de 1-5 mol/L, typiquement 2-3 mol/L est approprié pour effectuer cette étape d'hydrolyse. On peut utiliser d'autres acides, mais on utilise de préférence l'acide chlorhydrique car sa base conjuguée, l'ion Cl⁻ , est chimiquement neutre vis-à-vis des réactions chimiques possibles avec d'autres constituants du mélange traité, comparée aux bases conjuguées de certains autres acides tels que sulfate ou acétate. Selon un mode d'exécution, on ajoute au milieu d'hydrolyse une proportion mineure, de l'ordre de 10-30% en poids, et de préférence 20-25% en poids, d'un solvant organique totalement miscible à l'eau, tel que l'éthanol ou le méthanol, pour améliorer le rendement. Selon un mode d'exécution, l'hydrolyse est effectuée à une température supérieure à la température ambiante, choisie entre 60°C et 95°C. Après l'étape d'hydrolyse, dont la durée est usuellement comprise entre une demi-heure et 3 heures, le mélange réactionnel peut être refroidi.

Puis le mélange réactionnel est extrait une ou plusieurs fois au moyen d'un solvant non miscible ou faiblement miscible à l'eau. On utilise de préférence un solvant organique légèrement polaire (typiquement 3 fois le volume de la phase aqueuse). Selon un mode d'exécution, le solvant d'extraction est l'acétate d'éthyle. Puis le solvant est évaporé sous pression réduite et l'extrait d'hydrolysat selon l'invention est ainsi obtenu.

La formulation topique, objet de l'invention, doit contenir l'extrait d'hydrolysat selon l'invention dans un vecteur dermatologiquement acceptable. Ce vecteur peut être choisi parmi les onguents, les pommades, les crèmes, les laits et lotions connus de l'homme du métier. La formulation comprend de préférence entre 0.05% et 1% (en masse) de l'extrait d'hydrolysat de *P. kurroa* dans une forme galénique favorisant la pénétration de composés organiques polaires.

Dans une variante d'exécution, la formulation topique objet de l'invention contient de plus un ligand agoniste ou un proligand du recepteur AhR choisi parmi le L-tryptophane et les dérivés du tryptophane métabolisables par les enzymes de phase I.

Dans une variante d'exécution de la formulation topique, on ajoute sous forme pure un ou plusieurs aglycones de l'extrait objet de l'invention.

### Exemples

### Exemple 1 : Préparation d'extrait d'hydrolysat de Picrorhiza kurroa selon l'invention

Le point de départ est une poudre, d'origine commerciale, Kutki de HerbsForever, par exemple, obtenue à partir du broyage des racines et du rhizome de la plante *Picrorhiza kurroa.*

Cette poudre est partiellement solubilisée dans un mélange comprenant 25% de méthanol et 75% d'acide chlorhydrique à 2.7 mol/L, dont le volume est de 10 ml par g de poudre. Le tout est chauffé une heure à 80°C, permettant l'hydrolyse des glycosides. Après retour à température ambiante, le mélange est extrait deux fois avec 3 volumes d'acétate d'éthyle. Le solvant est évaporé sous pression réduite et l'extrait enrichi en composés monophénoliques est ainsi obtenu.

Exemple 2, comparatif : Préparation d'un extrait de *Picrorhiza kurroa* sans hydrolyse acide Le point de départ est la même poudre, obtenue à partir du broyage des racines et du rhizome de la plante *Picrorhiza kurroa,* que dans l'exemple 1.

L'extraction est effectuée par un solvant organique polaire, i.e. le méthanol ou l'éthanol. La poudre est partiellement solubilisée dans un mélange comprenant 10 ml de méthanol par gramme de poudre. Le tout est chauffé une heure à 40°C, permettant l'extraction de composés organiques polaires.

### Analyse hromatographique des deux extraits de Picrorhiza kurroa

Les deux extraits de *Picrorhiza kurroa* sont analysés par HPLC-UV (Agilent) sur une colonne C18 Nucleodur^{®} Pyramid 3 µm (Macherey-Nagel) au moyen d'un gradient linéaire de phases mobiles à flux constant (Tableau 1) :

**Tableau 1. Gradient de concentration pour la phase mobile de l'analyse par HPLC des extraits de P. kurroa. A : acide acétique 0.2% (aqueux); B : acétonitrile; C : méthanol. La détection est réalisée par mesure simultanée de l'absorbance aux longueurs d'onde suivantes : 260 nm, 275 nm, 310 nm, 322 nm.**

| Temps (min) | % A | % B | % C | Flux (ml / min) |
|---|---|---|---|---|
| 0 | 50 | 50 | 0 | 0.800 |
| 9 | 50 | 50 | 0 | 0.800 |
| 12 | 0 | 100 | 0 | 0.800 |
| 20 | 0 | 100 | 0 | 0.800 |
| 22 | 0 | 0 | 100 | 0.800 |
| 35 | 0 | 0 | 100 | 0.800 |

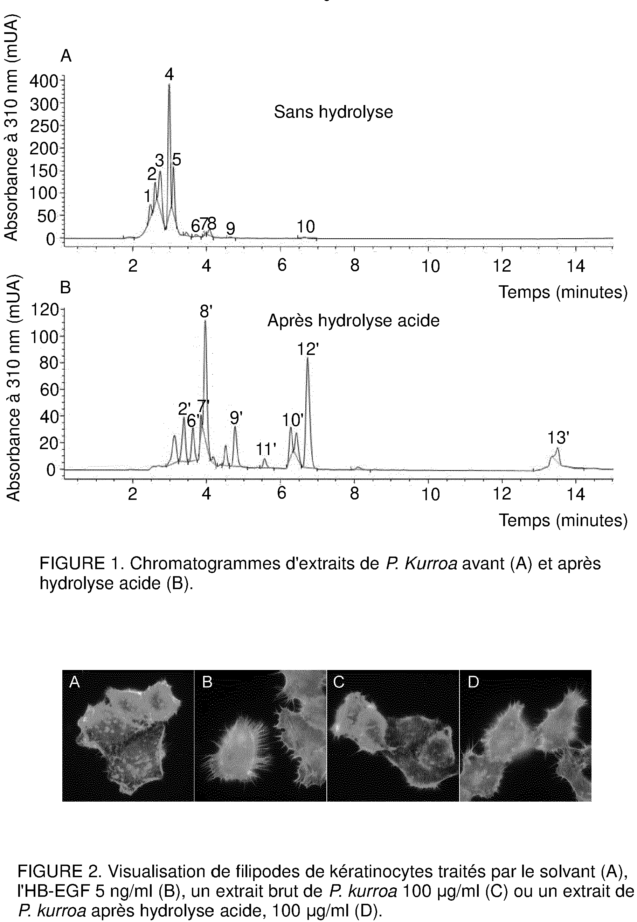

Les chromatogrammes des deux extraits de *Picrorhiza kurroa* sans et après hydrolyse acide montrent (Figure 1) :
- sur la figure 1A, sans hydrolyse : on observe des grands pics précoces (3, 4, 5), dont les spectres d'absorption correspondent à ceux de pics plus tardifs qui apparaissent dans le chromatogramme de l'extrait après hydrolyse acide (Figure 1B). Il s'agit entre autre des acides caféique, vanillique, coumarique et férulique, ainsi que certains de leurs dérivés ;
- sur la figure 1B (après hydrolyse), les pics 3, 4, 5 sont remplacés par de nouveaux pics plus tardifs avec des spectres d'absorption identiques : cela s'explique par l'hydrolyse de glycosides ayant libéré des aglycones, responsables des spectres d'absorption obtenus car les parties glycosidiques (sucres) des glycosides n'absorbent pas la lumière au-delà de 220 nm; les aglycones étant moins polaires que les glycosides, ils sont davantage retenus par la colonne de séparation et atteignent le détecteur plus tard.

Les pics 3, 4, 5 (A) sortent plus tôt - et sont donc plus polaires (glycosides) - et ont les mêmes spectres d'absorption que les pics 6-6', 8-8', 10-10'-13', respectivement (A, B). Il s'agit vraisemblablement de glycosides dont les aglycones apparaissent surtout après hydrolyse acide. Identification des pics : 2, 2' : acide caféique; 6, 6' : acide vanillique; 7, 7' : acide coumarique; 8, 8' : acide férulique; 9, 9' : apocynine, 10, 10' : acide cinnamique. Le pic 11' a le même spectre d'absorption que l'acide vanillique (6, 6'), le pic 12' a le même spectre que l'acide férulique (8, 8'), et le pic 13' a le même spectre que les pics 5, 10 et 10'.

### Exemple 3 : Propriétés biologiques in vitro de l'extrait d'hydrolysat de P. kurroa

- Le tableau 2 indique l'intensité de la formation des filopodes (nombre et surface) *in vitro* dans quatre cultures de kératinocytes dans quatre conditions opératoires, après traitement par :
- un solvant,
- HB-EGF (*heparin-binding epidermal growth factor*),
- extrait de *Picrorhiza kurroa* obtenu sans hydrolyse, et
- extrait de *Picrorhiza kurroa* obtenu suite à une hydrolyse.

L'HB-EGF (contrôle positif) a un effet intense, l'extrait hydrolysé de *Picrorhiza kurroa* a un effet moyen, et le solvant et l'extrait non hydrolysé n'ont pas d'effet.

L'essai est effectué au moyen d'une culture de kératinocytes humains répartie en quatre lots. Les cellules sont traitées 1 h avec les produits à tester, fixées au formol 4%, puis colorées avec de l'α-phalloïdine conjuguée à la rhodamine (l'α-phalloïdine a une grande affinité pour les filaments d'actine, et sa conjugaison à la rhodamine rend les filaments d'actine fluorescents [540 nm; 565 nm]). La morphologie des cellules est ensuite analysée au microscope en fluorescence (figure 2).
- A, solvant, négatif (pas de stimulation);
- B, HB-EGF (*heparin-binding epidermal growth factor*), facteur trophique de référence de l'épiderme, 5 ng/ml;
- C, extrait de *Picrorhiza kurroa* obtenu sans hydrolyse, 100 µg/ml;
- D, extrait de *Picrorhiza kurroa* obtenu suite à une hydrolyse, 100 µg/ml.

Le tableau 2 montre la production de filopodes.

**Tableau 2. Evaluation visuelle de la production de filopodes (échelle de 0 à 3).**

| Solvant | HB-EGF (5 ng/ml) | Extrait brut de *P. kurroa* obtenu sans hydrolyse (100 µg/ml) | Extrait de *P. kurroa* obtenu suite à une hydrolyse (100 µg/ml) |
|---|---|---|---|
| 0 | 3 | 0-1 | 1-2 |

### Exemple 4 : Etude clinique sur des sujets présentant une peau fragilisée : compte des purpuras séniles.

Les premiers signes cliniques de cette fragilité sont le plus souvent des *hématomes superficiels* qui semblent spontanés mais qui sont en fait dus à des traumatismes minimes de la vie courante qui jusque-là étaient tolérés. On les appelle *purpuras séniles.* Ils constituent un bon moyen de tester l'effet protecteur d'une préparation topique lorsqu'ils diminuent en nombre et fréquence lors de l'application prolongée de ladite préparation topique.

Dans cette optique une composition destinée à une application cutanée topique (voir Tableau 3), selon l'exemple 4 a été appliquée à onze patients atteints de dermatoporose sur la face postérieure des avant-bras - site privilégié des purpuras séniles - pendant plusieurs semaines (Tableau 4). La préparation était appliquée chaque soir pendant au moins 12 semaines. Le nombre des purpuras séniles était relevé chaque semaine (et la zone photographiée par le patient sur son téléphone portable). Le tableau 4 montre qu'avant le début de l'application de la préparation topique, sur les 22 sites analysés, le nombre des purpuras séniles présents était en moyenne de 6.8. Ces nombres moyens diminuaient chaque mois à 4.2 au bout d'un mois, puis à 2.2 au bout de deux mois, puis à 1.6 au bout de trois mois. Ces observations indiquent un effet remarquable de la préparation.

**Tableau 3. Composition de la formule topique selon l'exemple 4.**

| |
|---|
| eau |
| huile de soja |
| glycérol |
| Span^{®}60 |
| Myrj 52 |
| triglycérides capryliques/capriques |
| vaseline |
| alcool cétylique |
| myristate d'isopropyle |
| monooléate de glycérol |
| extrait de *P. kurroa* obtenu suite à une hydrolyse |
| α-tocophérol |
| éthanol |
| méthylparabène |
| hyaluronate de sodium |
| gomme xanthane |
| all-*trans*-rétinal |

**Tableau 4. Purpuras séniles.**

| | **Nombre de purpuras séniles** | | | |
|---|---|---|---|---|
| **Site analysé** | **Avant** | **Après 1 mois** | **Après 2 mois** | **Après 3 mois** |
| Avant-bras G | 4 | 3 | 2 | 1 |
| Avant-bras D | 5 | 3 | 1 | 1 |
| Avant-bras G | 4 | 3 | 2 | 1 |
| Avant-bras D | 3 | 1 | 1 | 1 |
| Avant-bras G | 10 | 6 | 4 | 3 |
| Avant-bras D | 9 | 7 | 5 | 2 |
| Avant-bras G | 4 | 3 | 2 | 1 |
| Avant-bras D | 3 | 2 | 2 | 2 |
| Avant-bras G | 5 | 2 | 2 | 2 |
| Avant-bras D | 3 | 3 | 1 | 1 |
| Avant-bras G | 9 | 7 | 4 | 2 |
| Avant-bras D | 10 | 6 | 3 | 3 |
| Avant-bras G | 20 | 10 | 3 | 3 |
| Avant-bras D | 11 | 7 | 2 | 4 |
| Avant-bras G | 4 | 2 | 1 | 1 |
| Avant-bras D | 3 | 1 | 1 | 1 |
| Avant-bras G | 8 | 3 | 2 | 1 |
| Avant-bras D | 12 | 6 | 3 | 1 |
| Avant-bras G | 3 | 2 | 1 | 1 |
| Avant-bras D | 2 | 2 | 1 | 1 |
| Avant-bras G | 8 | 5 | 2 | 1 |
| Avant-bras D | 9 | 8 | 3 | 2 |
| **Nombre total de purpura** | **149** | **92** | **48** | **36** |
| **Nombre de sites testés** | **22** | **22** | **22** | **22** |
| **Nombre moyen de purpuras** | **6.8** | **4.2** | **2.2** | **1.6** |

### Exemple 5: Etude clinique sur des sujets présentant une peau fragilisée : analyse de l'épaisseur cutanée par ultrasonographie.

La dermatoporose se caractérise par une perte de matrice extracellulaire et cellulaire. Ainsi, tout comme on mesure l'ostéo-densimétrie pour le diagnostic de l'*ostéo*porose, il est possible d'évaluer la peau de la dermatoporose par ultrasonographie, par exemple à l'aide de l'instrument d'ultrasonographie I-200 d'Epi Scan SRL. Cet examen permet d'objectiver la perte de matrice extracellulaire et cellulaire cutanée qui se traduit par une diminution de l'épaisseur d'une zone dermo-epidermique définie. Ainsi, l'épaisseur mesurée sur les faces dorsales des avant-bras est de 1.5 mm à l'état normal, mais peut être diminuée à moins de 0.5 mm, soit une perte de 67% dans la peau de dermatoporose. Il a été rapporté que des traitements topiques efficaces à base de retinaldéhyde et de fragments d'acide hyaluronique pouvaient induire un gain de 20 à 30 % chez des patients atteints de dermatoporose (Kaya et al., 2018).

Dans cette optique une composition destinée à une application cutanée topique, selon l'invention a été appliquée par 8 sujets atteints de dermatoporose (intrinsèque chez 4 sujets, et après usage de corticostéroïdes chez 4 autres sujets), sur la face postérieure des avant-bras, la face antérieure des jambes et le décolleté - sièges classiques de la dermatoporose - pendant plusieurs mois (jusqu'à 6 mois, Tableau 5). La préparation était appliquée chaque soir pendant au moins 8 semaines. Les mesures étaient réalisées au moyen d'un Episcan I-200 ultrsound scanner. Le tableau 5 montre que la moyenne d'augmentation de l'épaisseur cutanée atteignait 14.7% au bout de deux mois, 19.3% au bout de trois mois, 38.6% au bout de quatre mois, 18.6% au bout de cinq mois et 30.1 % au bout de six mois. Ces résultats sont comparables à ceux obtenus avec des traitements topiques efficaces à base de rétinal et de fragments d'acide hyaluronique et ils indiquent une activité de la préparation selon l'invention.

**Tableau 5. Evaluation de l'épaisseur épidermique.**

| AVBD : avant-bras droit, AVBG : avant-bras gauche, JD : jambe droite, JG : jambe DC : décolleté. gauche, | | | | | | |
|---|---|---|---|---|---|---|
| | | **Pourcentage d'augmentation en fonction du temps d'application** | | | | |
| **Patient** | **Localisation** | **2 mois** | **3 mois** | **4 mois** | **5 mois** | **6 mois** |
| 1 | AVBD | 13 | | | 15 | |
| | AVBG | 8 | | | 21 | |
| | JD | 7 | | | 21 | |
| | JG | 7 | | | 17 | |
| | DC | 3 | | | 10 | |
| 2 | AVBD | | 27 | | | 53 |
| | AVBG | | 26 | | | 51 |
| | JD | | 15 | | | 27 |
| | JG | | 15 | | | 38 |
| | DC | | 13 | | | 39 |
| 3 | AVBD | | -17 | | | -7 |
| | AVBG | | 32 | | | 36 |
| 4 | AVBD | | 31 | | | |
| | AVBG | | 32 | | | |
| 5 | AVBD | 2 | | | | |
| | AVBG | 1 | | | | |
| | JD | 6 | | | | |
| | JG | -2 | | | | |
| | DC | 9 | | | | |
| 6 | AVBD | 37 | | 45 | | |
| | AVBG | 36 | | 41 | | |
| | JD | 33 | | 42 | | |
| | JG | 30 | | 35 | | |
| | DC | 24 | | 30 | | |
| 7 | AVBD | 22 | | | | 28 |
| | AVBG | 5 | | | | 14 |
| | JD | 29 | | | | 32 |
| | JG | 10 | | | | 20 |
| 8 | AVBD | 13 | | | 22 | |
| | AVBG | 16 | | | 24 | |
| **Total** | | **309** | **174** | **193** | **130** | **331** |
| **Nombre de sites testés** | | **21** | **9** | **5** | **7** | **11** |
| **Augmentation moyenne** | | **14.7** | **19.3** | **38.6** | **18.6** | **30.1** |

### References

[1] Saurat JH. Quand la peau devient insuffisante. Méd Hyg. 2004; 476.
[2] Kaya G, Kaya A, Sorg O, Saurat JH. Dermatoporosis: a further step to recognition. J Eur Acad Dermatol Venereol. 2018;32; 189-191.
[3] Humbert P, Fanian F, Lihoreau T, Jeudy A, Pierard GE. Bateman purpura (dermatoporosis): a localized scurvy treated by topical vitamin C - double-blind randomized placebo-controlled clinical trial. J Eur Acad DermatolVenereol. 2018;32; 323-328.
[4] Kaya G, Saurat JH. Dermatoporosis: a chronic cutaneous insufficiency/fragility syndrome. Clinicopathological features, mechanisms, prevention and potential treatments. Dermatology. 2007;215; 284-294.
[5] Kluger N. Dermatoporosis and vitamin C deficiency. J Eur Acad Dermatol Venereol. 2018.
[6] Kluger N, Schaan K, Girard C, Guillot B, Dereure O. Hématomes multiples sur dermatoporose et carence en vitamine C. Nouv Dermatol. 2010;29; 371-373.
[7] Saurat JH. Dermatoporosis. The functional side of skin aging. Dermatology. 2007;215; 271-272.
[8] Mengeaud V, Dautezac-Vieu C, Josse G, Vellas B, Schmitt AM. Prevalence of dermatoporosis in elderly French hospital in-patients: a cross-sectional study. Br J Dermatol. 2012;166; 442-443.
[9] WO 2005/082327 A1
[10] WO 2009/093207
[11] Krupashree K, Kumar KH, Rachitha P, Jayashree GV, Khanum F. Chemical composition, antioxidant and macromolecule damage protective effects of Picrorhiza kurroa Royle ex Benth. South Afr J Bot. 2014;94; 249-254.

## Revendications

1. Un extrait d'une plante du genre *Picrorhiza,* choisie parmi *Picrorhiza kurroa, Picrorhiza scrophulariiflora* et *Neopicrorhiza scrophulariiflora,* obtenu par extraction par un premier solvant organique d'un hydrolysat aqueux acide d'éléments de la partie souterraine de la plante, lesdits éléments étant choisis parmi les rhizomes et les racines.

2. Extrait selon la revendication 1, **caractérisé en ce que** ladite plante est *Picrorhiza kurroa.*

3. Extrait selon la revendication 1 ou 2, **caractérisé en ce que** l'étape d'hydrolyse est effectuée en milieu aqueux comprenant 10 à 30% en volume d'un deuxième solvant organique totalement miscible à l'eau et 1 à 5 M/l de HCl.

4. Extrait selon l'un des revendications précédentes, **caractérisé en ce que** le premier solvant est l'acétate d'éthyle.

5. Mélange comprenant plusieurs extraits selon l'une des revendications précédentes.

6. Composition, **caractérisée en ce qu'**elle comprend :
- un extrait selon l'une des revendications 1 à 4, ou
- un mélange selon la revendication 5
dans un vecteur dermatologiquement acceptable.

7. Composition selon la revendication précédente, dans laquelle l'extrait est un extrait de *Picrorhiza kurroa* à une concentration comprise entre 0.05% et 1% (en masse).

8. Composition selon la revendication 6 ou 7, comprenant un ou plusieurs aglycones de l'extrait ou du mélange d'extraits, en particulier l'acide vanillique (par exemple entre 0.1% et 0.5% en masse) et/ou l'acide férulique (par exemple entre 0.1% et 0.5% en masse) et/ou l'acide caféique (par exemple entre 0.1% et 0.5% en masse) et/ou l'acide coumarique (par exemple entre 0.1% et 0.5% en masse) et/ou l'apocynine (par exemple entre 0.5% et 1% en masse).

9. Composition selon l'une des revendications 6 à 8, contenant de plus un ligand agoniste ou un proligand du recepteur AhR choisi parmi le L-tryptophane et les dérivés du tryptophane métabolisables par les enzymes de phase I.

10. Composition selon l'une des revendications 6 à 9, **caractérisée en ce que** la composition est destinée à une application cutanée topique.

11. Utilisation cosmétique d'une composition selon l'une des revendications précédentes 6 à 10, en formulation topique, pour traiter un vieillissement de la peau.

12. Extrait selon l'une des revendications 1 à 4 ou mélange selon la revendication 5 ou composition selon l'une des revendications précédentes 6 à 10, pour utilisation comme médicament, notamment pour utilisation dans le traitement et la prévention de la dermatoporose intrinsèque ou secondaire à l'usage de corticostéroïdes et/ou pour utilisation dans un traitement par administration locale.

13. Procédé d'obtention d'un extrait d'une plante du genre *Picrorhiza,* choisie parmi *Picrorhiza kurroa, Picrorhiza scrophulariiflora* et *Neopicrorhiza scrophulariiflora* selon l'une des revendications 1 à 4 ou 12 ou d'un mélange d'extraits selon la revendication 5, le procédé comprenant les étapes suivantes :
- broyer des racines et/ou des rhizomes de plante pour obtenir une poudre,
- mettre la poudre dans une solution aqueuse d'un acide,
- réaliser une extraction du mélange au moyen d'un solvant non miscible ou faiblement miscible à l'eau,
- évaporer le solvant.
